Europäisches Patentamt

European Patent Office

Office européen des brevets

(11) Veröffentlichungsnummer: **0 010 757**
**B1**

(12) **EUROPÄISCHE PATENTSCHRIFT**

(45) Veröffentlichungstag der Patentschrift:
**10.11.82**

(21) Anmeldenummer: **79104236.9**

(22) Anmeldetag: **31.10.79**

(51) Int. Cl.³: **A 61 M 25/00,** A 61 B 6/00

(54) **Katheter mit Röntgenkontraststreifen.**

(30) Priorität: **03.11.78 DE 7832684 U**

(43) Veröffentlichungstag der Anmeldung:
**14.05.80 Patentblatt 80/10**

(45) Bekanntmachung des Hinweises auf die Patenterteilung:
**10.11.82 Patentblatt 82/45**

(84) Benannte Vertragsstaaten:
**AT BE CH DE FR GB IT LU NL SE**

(56) Entgegenhaltungen:
**DE-A-1 491 652**
**DE-A-1 965 487**
**DE-A-2 553 100**
**DE-B-1 108 860**
**DE-C-823 320**
**GB-A-1 178 285**
**US-A-2 212 334**
**US-A-3 070 132**
**US-A-3 302 635**
**US-A-3 483 859**
**US-A-3 618 614**
**US-A-4 038 519**

(73) Patentinhaber: **Intermedicat GmbH, Gerliswilstrasse 45, CH-6020 Emmenbrücke (CH)**

(72) Erfinder: **Herlitze, Gerd, Binsdorferstrasse 4, Baunatal 7 (DE)**
Erfinder: **Werner, Hans-Theo, Parkstrasse 1, Edermünde-Grifte (DE)**

(74) Vertreter: **von Kreisler, Alek, Dipl.-Chem. et al, Deichmannhaus am Hauptbahnhof, D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Katheter mit Röntgenkontraststreifen

Die Erfindung bezieht sich auf einen Katheter zur einmaligen Verwendung mit in Längsrichtung des Katheters wendelförmig angeordneten Röntgenkontraststreifen.

Ein bekannter Katheter (DE-A1-2 553 100) ist mit einem für Röntgenstrahlen undurchlässigen Streifen versehen, der in Längsrichtung des Katheters gerade verläuft. Ein solcher gerader Röntgenkontraststreifen ist schmal und lässt sich am Röntgenschirm nicht eindeutig erkennen, weil parallel zu dem in der Vene vorgeschobenen Katheter auch Gefässe, Stränge und Organe verlaufen können, die wegen ihrer hohen Dichte ebenfalls röntgenologisch sichtbar werden, so dass sich der Katheter von dem Umfeld, in dem er verlegt ist, nicht differenziert abhebt. Die Darstellung wird noch undeutlicher, wenn der Röntgenkontraststreifen sich in bezug auf den Röntgenschirm auf der Rückseite des Katheters befindet. Ferner lässt sich die jeweilige Verlegelänge dieses Katheters röntgenologisch nicht ermitteln.

Um die vorgeschobene Länge eines Röhrchens zur Speiseröhrenbehandlung (GB-A-1 178 285) zu veranschaulichen, sind zusätzlich zu einem in Längsrichtung gerade verlaufenden Röntgenkontraststreifen numerierte Ringmarkierungen vorgesehen. Da diese nicht aus Röntgenkontrastmaterial bestehen, sind sie nur extrakorporal sichtbar. Für einen langen Katheter ist eine solche Anzeige zu ungenau und für den Chirurgen zu unbequem.

Ein weiterer bekannter Katheter (US-A-3 070 132) ist mit einem einzigen schraubenförmig verlaufenden Röntgenkontraststreifen ausgestattet. Ein einziger schraubenförmiger Röntgenkontraststreifen hebt sich jedoch von ebenfalls röntgenologisch sichtbaren Organen genausowenig ab, wie ein gerader Röntgenkontraststreifen, so dass auch er schlecht erkennbar ist. Ausserdem fehlen Anhaltspunkte für die verlegte Länge des Katheters.

Der Erfindung liegt die Aufgabe zugrunde, einen Katheter mit Röntgenkontraststreifen so auszubilden, dass seine Lage unabhängig von anderen röntgenologisch dargestellten Organen differenziert und zuverlässig am Röntgenschirm erkennbar ist und dass dabei auch die verlegte Länge des Katheters eindeutig beurteilt werden kann.

Diese Aufgabe wird gelöst durch zwei oder mehrere in Längsrichtung des Katheters wendelförmig und versetzt angeordnete Röntgenkontraststreifen, die in der Projektion bei der Röntgendarstellung eine in axialer Richtung des Katheters aneinandergereihte Kette von Schnittpunkten ergeben.

Ein solcher zur einmaligen Verwendung, insbesondere als Venenkatheter, geeigneter Katheter lässt sich aufgrund der in jeder Lage des Katheters auf dem Röntgenschirm exakt erkennbaren Kette von Schnittpunkten am Röntgenschirm deutlich verfolgen, weil die Schnittpunkte nicht mit röntgenologisch ebenfalls dargestellten Organen verwechselt werden können, so dass eine differenzierte Beurteilung der Katheterlage möglich ist. Die Darstellung der sich in der Projektion an Schnittpunkten kreuzenden wendelförmigen Röntgenkontraststreifen wird durch die doppelte Materialdicke der Röntgenkontraststreifen in den Schnittpunkten verbessert. Die Schnittpunkte ergeben sich jeweils durch die in der projizierten Ebene kreuzweise verlaufenden Röntgenkontraststreifen, deren Anzahl pro Längeneinheit in axialer Richtung des Katheters vom gegenseitigen Abstand der Röntgenkontraststreifen und deren Steigung bestimmt wird. Die auf diese Weise erzielte röntgenologisch erkennbare Teilung des Katheters kann ein Mass für die verlegte Länge des Katheters bilden und dient als Orientierungshilfe beim Vorschieben des Katheters und bei der Beurteilung seiner Lage.

Die gegenseitigen Abstände der mindestens zwei Röntgenkontraststreifen können regelmässig oder unregelmässig sein. Durch entsprechende Wahl der Wendelsteigung der Anzahl der Röntgenkontraststreifen ergeben der Abstand und die Anzahl der projizierten Schnittpunkte eine metrische Teilung.

Der Querschnitt der Röntgenkontraststreifen innerhalb der Katheterwandung weist eine beliebige Grösse und Form auf. Es ist lediglich wesentlich, dass der Katheter mit Röntgenkontraststreifen eine homogene, in sich geschlossene, glatte Gesamtoberfläche aufweist, durch die eine Thrombenbildung vermieden wird.

Der Katheter wird aus transparentem Material gefertigt. Die Röntgenkontraststreifen bestehen aus einem für Röntgenstrahlen undurchlässigen Werkstoff.

Die Erfindung wird im Folgenden unter Bezugnahme auf die Zeichnungen erläutert. Es zeigt:

Fig. 1 eine Draufsicht auf einen Katheter mit zwei parallel verlaufenden Röntgenkontraststreifen in der projizierten Ebene, und

Fig. 2 einen Schnitt durch den Katheter nach Fig. 1 längs der Linie A–A.

Durch die wendelförmige, versetzte Anordnung von zwei Röntgenkontraststreifen 2 und 3 kreuzen sich in der Projektion ihre auf der Rückseite des Katheters befindlichen Abschnitte 4 und 5 mit denen auf der Vorderseite vorhandenen gegenläufigen Abschnitten des jeweils anderen Röntgenkontraststreifens 2 bzw. 3, und es entstehen sich in regelmässigen Abständen wiederholende Schnittpunkte 1. Der Katheterschlauch besteht aus transparentem Werkstoff, so dass das ihn durchströmende Material im extern liegenden Teil des Katheters sichtbar ist.

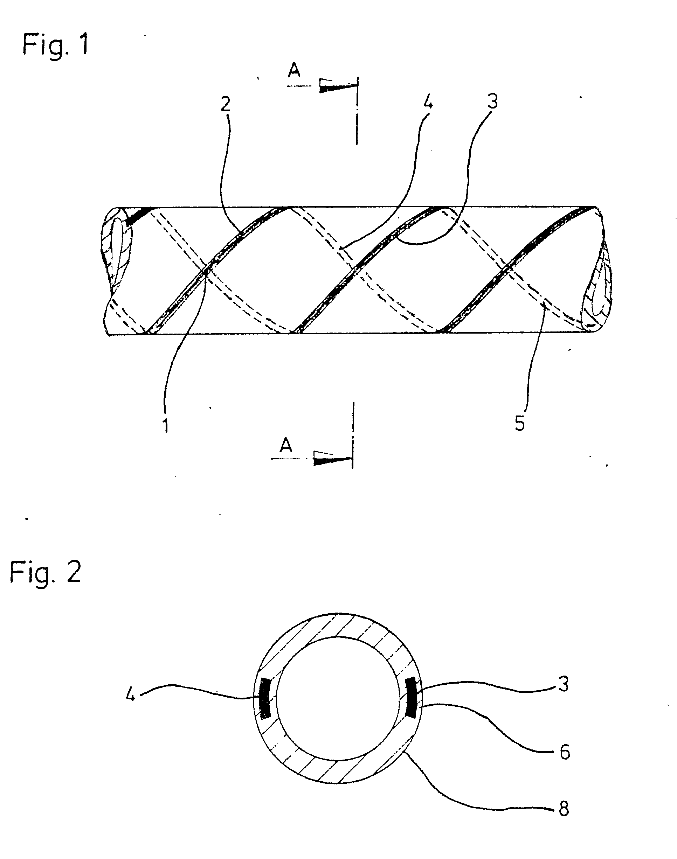

Der in Fig. 2 gezeigte Querschnitt verläuft durch die in Fig. 1 gezeigten projizierten Schnittpunkte 1, und es ist die doppelte Materialdicke erkennbar, die sich in der Projektion durch Kreuzung der Abschnitte der Kontraststreifen 2 bzw. 3 auf der Vorderseite des Katheters mit den Abschnitten 4 bzw. 5 auf der Rückseite des Katheters ergibt.

Die Röntgenkontraststreifen 2 und 3 sind in das Material der Wand 6 versenkt so eingebettet, dass der Katheter eine in sich geschlossene, homogene und glatte Gesamtoberfläche 8 aufweist.

## Patentansprüche

1. Katheter zur einmaligen Verwendung mit in Längsrichtung des Katheters wendelförmig angeordnetem Röntgenkontraststreifen (2, 3), gekennzeichnet durch zwei oder mehrere in Längsrichtung des Katheters wendelförmig und versetzt angeordnete Röntgenkontraststreifen (2, 3), die in der Projektion bei der Röntgendarstellung eine in axialer Richtung des Katheters aneinandergereihte Kette von Schnittpunkten (1) ergeben.

2. Katheter nach Anspruch 1, dadurch gekennzeichnet, dass die gegenseitigen Abstände der mindestens zwei Röntgenkontraststreifen (2, 3) regelmässig oder unregelmässig sind.

3. Katheter nach Anspruch 1, dadurch gekennzeichnet, dass durch entsprechende Wahl der Wendelsteigung und der Anzahl der Röntgenkontraststreifen (2, 3) der Abstand und die Anzahl der projizierten Schnittpunkte (1) eine metrische Teilung ergeben.

4. Katheter nach einem der Ansprüche 1 bis 3, dadurch gekennzeichnet, dass der Querschnitt der Röntgenkontraststreifen (2, 3) innerhalb der Katheterwandung (6) eine beliebige Grösse und Form aufweist.

5. Katheter nach einem der Ansprüche 1 bis 4, dadurch gekennzeichnet, dass der Katheter aus transparentem Material gefertigt ist und dass die Röntgenkontraststreifen (2, 3) aus einem für Röntgenstrahlen undurchlässigen Werkstoff bestehen.

## Claims

1. A disposable catheter having an X-ray contrast strip (2, 3) arranged spiral-like in the longitudinal direction of the catheter, characterized by two or more X-ray contrast strips (2, 3) arranged spiral-like and in staggered form in the longitudinal direction of the catheter which strips, when X-rayed appear in the projection as a chain of points of intersection (1) successively arranged in axial direction of the catheter.

2. Catheter according to claim 1, characterized in that the mutual distances of at least two X-ray contrast strips (2, 3) are regular or irregular.

3. Catheter according to claim 1, characterized in that by a corresponding selection of the spiral pitch and the number of the X-ray contrast strips (2, 3) the distance and the number of the projected points of intersection (1) result in a metric graduation.

4. Catheter according to one of the preceding claims 1 to 3, characterized in that the cross section of the X-ray contrast strips (2, 3) within the catheter walls (6) is optional as to size and shape.

5. Catheter according to one of the preceding claims 1 to 4, characterized in that the catheter is made of transparent material and that the X-ray contrast strips (2, 3) are of a material impermeable to X-rays.

## Revendications

1. Cathéter pour usage unique et comportant une bande de contraste aux rayons X (2, 3) disposée en forme de spirale dans la direction longitudinale du cathéter, caractérisé en ce qu'il est prévu deux ou plusieurs bandes de contraste aux rayons X (2, 3) disposées de façon décalée en forme de spirale dans la direction longitudinale du cathéter et qui définissent en projection lors de la représentation radiographique une chaîne de points d'intersection (1) disposés en rangée dans la direction axiale du cathéter.

2. Cathéter selon la revendication 1, caractérisé en ce que les espacements mutuels des bandes de contraste aux rayons X (2, 3) au moins au nombre de deux sont réguliers ou irréguliers.

3. Cathéter selon la revendication 1, caractérisé en ce que, par un choix correspondant de la pente de la spirale et du nombre de bandes de contraste aux rayons X (2, 3), l'espacement et le nombre des points d'intersection projetés (1) établissent une division métrique.

4. Cathéter selon l'une quelconque des revendications 1 à 3, caractérisé en ce que la section droite des bandes de contraste aux rayons X (2, 3) à l'intérieur de la paroi (6) du cathéter présente une grandeur et une forme quelconques.

5. Cathéter selon l'une quelconque des revendications 1 à 4, caractérisé en ce que le cathéter est formé d'un matériau transparent et en ce que les bandes de contraste aux rayons X (2, 3) sont formées d'une matière opaque aux rayons X.

Fig. 1

Fig. 2